# EUROPEAN PATENT SPECIFICATION

(11) **EP 1 647 603 B1**
(45) Date of publication and mention of the grant of the patent: **17.01.2007**
(21) Application number: 06000681.4
(22) Date of filing: 29.07.2003
(51) Int. Cl.: C12Q 1/68

(54) **Gene assay method for predicting glaucoma onset risk**
Genetisches Verfahren zur Vorhersage des Glaukomarisikos
Test génétique de prédiction des risques de glaucome

(30) Priority: 02.08.2002 JP 2002226612
(43) Date of publication of application: 19.04.2006
(62) Divisional of application: 03447201.9
(73) Proprietor: SYSMEX CORPORATION, Kobe-shi, Hyogo 651-0073 (JP)
(72) Inventor: Kouchi, Yasuhiro, c/o Sysmex Corporation, Kobe-shi Hyogo 651-0073 (JP); Masago, Akinori, c/o Sysmex Corporation, Kobe-shi Hyogo 651-0073 (JP); Takahata, Takayuki, c/o Sysmex Corporation, Kobe-shi Hyogo 651-0073 (JP)
(74) Representative: De Clercq, Ann G. Y.

(56) References cited:
- WO-A-03/056037
- REZAIE T ET AL: "Adult-onset primary open-angle glaucoma caused by mutations in optineurin" SCIENCE, AMERICAN ASSOCIATION FOR THE ADVANCEMENT OF SCIENCE,, US, vol. 295, 8 February 2002 (2002-02-08), pages 1077-1079, XP002961567
- REZAIE T. ET AL.: "Adult-onset Primary Open-Angle Glaucoma caused by Mutations in Optineurin: Supplementary Web Material" SCIENCE ONLINE, vol. 295, 8 February 2002 (2002-02-08), XP002370043 Retrieved from the Internet: URL:HTTP://WWW.SCIENCEMAG.ORG/CGI/CONTENT/ FULL/295/5557/1077/DC1> [retrieved on 2006-02-28]

### SEQUENCE LISTING

<110> Sysmex Corporation
<120> Gene assay method for predicting glaucoma onset risk
<130> SYS-004-EP-DIV1
<150> JP P2002-226612
   <151> 2002-08-02
<160> 40
<170> PatentIn version 3.1
<210> 1
   <211> 1734
   <212> DNA
   <213> Homo sapiens
<400> 1
   atgtcccatc aacctctcag ctgcctcact gaaaaggagg acagccccag tgaaagcaca      60
   ggaaatggac ccccccacct ggcccaccca aacctggaca cgtttacccc ggaggagctg     120
   ctgcagcaga tgaaagagct cctgaccgag aaccaccagc tgaaagaagc catgaagcta     180
   aataatcaag ccatgaaagg gagatttgag gagctttcgg cctggacaga gaaacagaag     240
   gaagaacgcc agtttttgga gatacagagc aaagaagcaa aagagcgtct aatggccttg     300
   agtcatgaga atgagaaatt gaaggaagag cttggaaaac taaaagggaa atcagaaagg     360
   tcatctgagg accccactga tgactccagg cttcccaggg ccgaagcgga gcaggaaaag     420
   gaccagctca ggacccaggt ggtgaggcta caagcagaga aggcagacct gttgggcatc     480
   gtgtctgaac tgcagctcaa gctgaactcc agcggctcct cagaagattc ctttgttgaa     540
   attaggatgg ctgaaggaga agcagaaggg tcagtaaaag aaatcaagca tagtcctggg     600
   cccacgagaa cagtctccac tggcacggca ttgtctaaat ataggagcag atctgcagat     660
   ggggccaaga attacttcga acatgaggag ttaactgtga gccagctcct gctgtgccta     720
   agggaaggga atcagaaggt ggagagactt gaagttgcac tcaaggaggc caaagaaaga     780
   gtttcagatt ttgaaaagaa aacaagtaat cgttctgaga ttgaaaccca gacagagggg     840
   agcacagaga aagagaatga tgaagagaaa ggcccggaga ctgttggaag cgaagtggaa     900
   gcactgaacc tccaggtgac atctctgttt aaggagcttc aagaggctca tacaaaactc     960
   agcaaagctg agctaatgaa gaagagactt caagaaaagt gtcaggccct tgaaaggaaa    1020
   aattctgcaa ttccatcaga gttgaatgaa aagcaagagc ttgtttatac taacaaaaag    1080
   ttagagctac aagtggaaag catgctatca gaaatcaaaa tggaacaggc taaaacagag    1140
   gatgaaaagt ccaaattaac tgtgctacag atgacacaca acaagcttct tcaagaacat    1200
   aataatgcat tgaaaacaat tgaggaacta acaagaaaag agtcagaaaa agtggacagg    1260
   gcagtgctga aggaactgag tgaaaaactg gaactggcag agaaggctct ggcttccaaa    1320
   cagctgcaaa tggatgaaat gaagcaaacc attgccaagc aggaagagga cctggaaacc    1380
   atgaccatcc tcagggctca gatggaagtt tactgttctg attttcatgc tgaaagagca    1440
   gcgagagaga aaattcatga ggaaaaggag caactggcat tgcagctggc agttctgctg    1500
   aaagagaatg atgctttcga agacggaggc aggcagtcct tgatggagat gcagagtcgt    1560
   catggggcga gaacaagtga ctctgaccag caggcttacc ttgttcaaag aggagctgag    1620
   gacagggact ggcggcaaca gcggaatatt ccgattcatt cctgccccaa gtgtggagag    1680
   gttctgcctg acatagacac gttacagatt cacgtgatgg attgcatcat ttaa          1734
<210> 2
   <211> 1166
   <212> DNA
   <213> Homo sapiens
<400> 2
   tgcaagctct gcctcccggg ttcacgccat tctcctgcct cagcctcccg agtagctggg      60
   actacaagcg cccaacacca agcccggcta attttttgta tttttagtag agacggggtt      120
   tcactgtgtt agccaggatg gtctcaatct cctgacctca tgatctgtcc gcctcggcct      180
   cccaaagtgc tgggattaca ggcgtgagcc accacgcccg gccctcattg taccctttta      240
   tacacccata cacacacacg cacacacaca catgcacaca tgcgcgtgca cacacacaca      300
   cacttttctg aagctacata tacctttttt gtttaaaagg aagaatcaaa aatgtccaaa      360
   atgtaactgg agagaaagtg ggcaactttt ggagtaagta ttagcaatcg ccaatgggtt      420
   tgtgggactc ccggggaccc cttgtggggc gggggacagc tctattttca acaggtgact      480
   tttccacagg aacttctgca atgtcccatc aacctctcag ctgcctcact gaaaaggagg      540
   acagccccag tgaaagcaca ggaaatggac ccccccacct ggcccaccca aacctggaca      600
   cgtttacccc ggaggagctg ctgcagcaga tgaaagagct cctgaccgag aaccaccagc      660
   tgaaaggtga gcagggctgg cccctgtgtg ccccattcat cctgggcctg caagaaatgc      720
   catccctttg cactaaggct tggtggtgag ctcccttctc cccgtttcca taggtggtag      780
   ctggtgggga agcacaggat ttagcatttg gcaaggctaa atctgttctg atttttactt      840
   ttggaaacag gtacaagtaa aaactgtgtg tatctcaagg aagtagcata atgatattta      900
   gcccattcaa aaggaaaaag aggctgggcg tggtggctca tgcctgtcat tccatcactt      960
   tgggaggccg aggcagaagg attgcttgag tacaggagtt caagaccagc ctgggcaaga     1020
   tggcaagacc tgatctctac aaaaaaatta aaaaaaaaaa aaaaaagctg ggcgtggtgg     1080
   tgcacgcctc tggtcctagc tactggggat gctgaggttg gaggattgct tgagcctggg     1140
   aagttggagc tgcagtgagc catgat                                          1166
<210> 3
   <211> 1203
   <212> DNA
   <213> Homo sapiens
<400> 3
   gcagtgagcc atgatcgtgc cactgcactt tagcctggat gacagagaga gaccctgact      60
   caaaaaaaaa aaaaaaaaaa ggaaaaagga agaaaggctg ctatggttcc agagttagtc     120
   ctatatatta ccttattaag agaaagcatc ctggtatctc aagatggctt tgggcaggac     180
   cagtatttga atctaggagt agtaagaact tccttagctc ctagtaacca tagatattta     240
   gatatttgtg ctgtagtggc ggtacccaaa tccactttat tttcttggga tttttaagga     300
   ctagaaatga tgttcatccc gctagtcttt tctgtaagca aaaaccactt cgtctttttg     360
   ctgctgaccc ttgggccaag gctaagcatg gcatctttca attcagagcc atgtggtcaa     420
   gtggactaga gggagatttg gttcatcaga tcaagtccac tttcctggtg tgtgactcca     480
   tcactctgaa cctcctgcag aagccatgaa gctaaataat caagccatga aagggagatt     540
   tgaggagctt tcggcctgga cagagaaaca gaaggaagaa cgccagtttt ttgagataca 600
   gagcaaagaa gcaaaagagc gtctaatggc cttgagtcat gagaatgaga aattgaagga 660
   agagcttgga aaactaaaag ggaaatcaga aaggtcatct gaggtgagca gaccgatcca 720
   ttgtgatgtt gttttttttt tttcccttga catttgcagt ggaatcttac gtgtctagac 780
   tcctagatca aaacctttca tggttcagtc tggattggtg ttttgcctgg tcttggaaga 840
   agtgcttttg ctgaaaagat tggttgccct attaagggtc atggataatc tcttttagaa 900
   gaaagaaatt tgtaaagctt tgaccgtact gattgtaggc aaaagaacag taaggttata 960
   aatcattgta ttgtattcat tatagatggt gcagatgggc ctctgcctag aaccaacaat 1020
   tgtttttagt ttgtctttga tataaaaaat atgtttaaaa aacccattac tcagaatttt 1080
   tacttgttga ccttgtctgt tctctcagtc taaaatggag attattcact ttacattttc 1140
   ctttttaaaa atgctttgga aaatgtcatg ttgtggtagg aggctatcgc attgccacag 1200
   atg 1203
<210> 4
   <211> 1183
   <212> DNA
   <213> Homo sapiens
<400> 4
   ttgtcctgcc tcagcctccc gagtagctgg gactacaggc gcccgccacc acgcccagct 60
   aatctttttg tatttttagt agagacgggg tttcactgtg ttagccagga tggtctccat 120
   ctcctgacct tcatgatccg cccacctcgg cctcccaaag tgctgggatt acaggcgtga 180
   gccaccacgc ctggcttggc tttttttttt ttttttttga gacagggtct tggcagtctt 240
   aaactcctgg gctcaggcag tcttcctgcc tcagcctccc aactaatggg gactacaggt 300
   gtgtgccact acacctggct aattattaaa ttttttgtaa agatgggggt cttgctatgt 360
   tgcccaggct ggtctcaaaa tcctggcctc aagggatcct cccacttcag cctcccagag 420
   ctctgcgatt aagggcatga gcccatggtg cccagcctta gtttgatctg ttcattcact 480
   ttactccttg tcatctccag gaccccactg atgactccag gcttcccagg gccgaagcgg 540
   agcaggaaaa ggaccagctc aggacccagg tggtgaggct acaagcagag aaggcagacc 600
   tgttgggcat cgtgtctgaa ctgcagctca agctgaactc cagcggctcc tcagaagatt 660
   cctttgttga aattaggatg gctgtgagtt tttggtttta tttttgtttt gagcaaacta 720
   taaagcctcc cctggaaaga tgaaacaaat accacttttt cttgtcaaca caagccaagg 780
   attgaggaaa ttccagtgta gcaaagataa attggctctc attttctaag tatagcataa 840
   tgcatgtaag ggttatcata gctaaaatgg aaaaatatta attacctttt atgatgaaag 900
   ctgtagtctt tttttttttc ttcatcatgt cctggcaaat tgaacatttt tgtgaccaga 960
   aaaggaaaaa acccacacga acatgaactt tctgtcattt ttcaaactag gtctcaaagc 1020
   tgtattccgc agttcactta agggagcgca aacatatttt cacaacagaa ccctcttttt 1080
   ttgttttgag acagagtctt actctgtctt cccggctgga atgcagtgat gtgatctcgg 1140
   ctcactgcac cctctgcctc cggggttcaa gagattctcg tgc 1183
<210> 5
   <211> 1074
   <212> DNA
   <213> Homo sapiens
<400> 5
   agtgacctgt ggtgcataca aatttctaat gggaaccaac ttggccaaga tggtgctttg      60
   tgaatctcat tcacagaaac tgcctctttt ttaactttac ctcagtgagt tctagcattt     120
   tgcattttaa aggaaggata tgtggagttg tcaccagctc tgtatgacct taaccttgag     180
   aaagagggaa ctgccaagga aagggaggag cagataagct ttcatgttta cagagtcagg     240
   tagaatgtgt atggcgagat gaaactgacc ttcacgcctt agctgggata tttataatcc     300
   cgacagggcg tgccaggtga ggggagggta cgtttccatt tcctctgagc caccccgttt     360
   aaacagtgca catctgaatg tttggaagct tccttgggtt gcatgtcaca aaaattcatc     420
   ttttgtcttt ttcttctttt gacaaagaat ttgtcttgta gacatattgt gttaaatccc     480
   ttgcatttct gttttcacag gaaggagaag cagaagggtc agtaaaagaa atcaagcata     540
   gtcctgggcc cacgagaaca gtctccactg gcacgtatgt gaaggaagac tcgggctgtc     600
   aggcagacag gctgggcagg ctcgtcactg ggtgcttgtc accggaggtc aaatgttgtg     660
   acctgaggaa gtaacttctt tatgatttat accaggatct ttccagaata tttggtttga     720
   atgctattta atgttgcagc tcaaactggc aaagattaaa aactgtttgg ttcctgtttg     780
   gctcacactg actgctctgt tctagtggtg tctcacctcc agcagatgaa aagtgaaagc     840
   aaactggttc tcaatcaagt caatgatttg ttcctaatca aagacatgtt tgctcattgg     900
   ttccccggtg ccatttgacc cagaccagcc tgcccagctt ccataagtga aatattttca     960
   ttttcttttc cctgctactt cccagttata agctggcatg gccaatactg gaacatcttt    1020
   tgtaacaatg actgatagca ctctcagtca ttgtgggtgt tgcctgaaag tgcc          1074
<210> 6
   <211> 1153
   <212> DNA
   <213> Homo sapiens
<400> 6
   atttctctgc tctcattatt tgaaaccaca agtgaaaaag gttttctccc cttgacttaa      60
   gctgtgatgg tctctgttaa cttggagaaa ggccagtggt ctgtacaatg tgcctttatc     120
   ttttgtctga ctgcagtccc ctttgagact agatctctgg aaagcttggc accttcagcc     180
   acggctgcct ctgctgaact gttccgtgag ttttgtggtg tggtgtgagg tacacagtga     240
   ctgtttggag gacgtgggtg tgtgcattgt aagctggcct ctccagagcc tcactgagtc     300
   tccacacctt ccctaggaag catggaggag cttggcactg ggggtcccag gaccagctgt      360
   gcttgttcac tagttgagaa ttagttggag aatgttctgg aaagcagttc ctttaagctg     420
   gtcccagtta tattgggtta ctctcttctt agtctttgga atttttctga tgaaaacctt     480
   ttaaccttta tactgaacag ggcattgtct aaatatagga gcagatctgc agatggggcc     540
   aagaattact tcgaacatga ggagttaact gtgagccagc tcctgctgtg cctaagggaa     600
   gggaatcaga aggtggagag acttgaagtt gcactcaagg aggccaaaga aaggtatgaa     660
   ataggttaac ttgaaatatg tgttttttta aaacagcttt cctgagatat aattaagata     720
   ccatacagtt cacccattta aagtatacat ttcagtgttt tttagaatat tccaggattg     780
   tgcaaccact gttactacaa tataatttta gaacattttt tcccccaaac agcactcact     840
   gtctgctcct ccaagcaatg tgctttctgt ctctatagat ttggccattc tagacatttc     900
   atataaatgg aattatacag tctgtggttt tttgtgactg gcttctttca cgtagcataa     960
   tgtttttgag gttcatctac aacgtagcat gtatcagtac ttccttttcc ttgctgaata    1020
   accttccatt gtctatatat acaacatttt gtttattcat tcatcagttg ataaacatta    1080
   gagttgttgc cactttttac ctattaggaa taatgctgct atgaacagtg tgtacaagtt    1140
   tttactggga tat                                                       1153
<210> 7
   <211> 1103
   <212> DNA
   <213> Homo sapiens
<400> 7
   ccacagtctc ttgtttcatt tggattggga cggctttcct gtggttatga tttggtgtta 60
   agaatggtgt tacttttttt gttgtcgttt attcggtgac ttttaaactt agctgtgtcc 120
   taaaaggaaa agtctttcct tctctaatga attcttatga atgagatacc atgttcatgg 180
   aacacacatg catccacatg tgtaaacaca aacaatttca aaaacattgc tgcataggac 240
   agttgcatgg aaacaaatgg tgttcaagat gagtttcact tgccttttac ctctgtgtgt 300
   atttgtctgt gaatcaattc tagccaattt taggatgaaa aataaaacta atgctaatat 360
   agtgaatgtg tagagatttt gaaaacccct gatcctttat cccaattgta aacaatgttc 420
   tttttagtac ttctgtaata attgctattt ctcttaaagc caaagagaaa gtaacttttc 480
   tatcttctgt gattttccag agtttcagat tttgaaaaga aaacaagtaa tcgttctgag 540
   attgaaaccc agacagaggg gagcacagag aaagagaatg atgaagagaa aggcccggag 600
   actgtgagtc ctaagattcc acggccacta ccacacccac acacacgaga gtagtccagc 660
   cactgaattc aaatcttgtg atgggttatt tgctttagaa atatagaaat catgttgata 720
   ttgaatatta tctatctatt ccttttatat gtccttgtcc tgctctgtgt caattgtagc 780
   gagatgtatt tcttttttgt tgttgttgtt ggagatggag tctcactctg tcgccaggct 840
   ggagtgcagt ggcacgatct cagctcactg caacctccgc ctcccaggtt caagcagttc 900
   tcctgcctca gcctcccaag tagctgggat tacaggtgcc cgtcaccacg cctggctaat 960
   ttttgtattt ttaatacaga cagggtttca ccatgttggc caggatggtc ttgatctctt 1020
   gacctcgtga tcctcccacc tcggcctccc agagtgctgg gattacagat atgagccact 1080
   gcgcccagct gcaagatgta ttt 1103
<210> 8
   <211> 1116
   <212> DNA
   <213> Homo sapiens
<400> 8
   acgaattcaa cagccagtag cagggaaata tggtctttca aggcatcaga aactcattta 60
   caaaaattat agagctgcca ggaaaaaggc tgcacaacaa aaatagttga gtaaactaga 120
   aacatacact gggaagagag tatgggggca agttgttagc tggatagata ggactgtgct 180
   ttgacacctc tgtggtctat gatctctgaa cctggaatag ggttcatttt aatagcgata 240
   aagtcattat cccagtgcat ccaaattgat tagttcatgc tttattagga aacagaagtt 300
   acccaaaact tagcaaacct aagtaccaag tatccaaaac attcttttcc tacacaatgt 360
   ttggggtatt gtcaaagttg gattgattca ccagccagtc ttaattggct actaatggtt 420
   cagcctgttt tctcctaaag aggtttgttt aatgtcagat gataattgta cagatatgtt 480
   tgggatttcc cgtatgatag gttggaagcg aagtggaagc actgaacctc caggtgacat      540
   ctctgtttaa ggagcttcaa gaggctcata caaaactcag caaagctgag ctaatgaaga      600
   agagacttca agaaaagtaa gaatgagaga gcaattttat cctcctttga aatatacatt      660
   tttacaaagt atactactat ataaaaacat agttttttaa ctatgttatg actaaaagaa      720
   aaatagacac ctaattaaaa tataaattca gaatatacta atgttccagt taatgtgtga      780
   gcatgaaata cttgtaagat ggggggttgg ggactggaga actttaattc tgccatttag      840
   gggcatttgt taaatgtacg agcctgggta agatctctac agtaaagctg tgagctagtt      900
   ttcctgttac tgacttaagc tgatgacatt gatgtgagta agcataaaga aagatgaaaa      960
   gagcataaag atcttgagtg acatttattt ggaaaaaggt caatttcaat ttgttatttc     1020
   aatcagttaa ttatttcagg ctaacatgta gattgagcgt ttggcatttg cttgtttctc     1080
   ttgatgtaag aagttaccca aaacttagca aaccta                               1116
<210> 9
   <211> 1150
   <212> DNA
   <213> Homo sapiens
<400> 9
   atgctttgtg catagctgtc atttatttgt attatattga aatcctcttt ccgatcttta    60
   agaagactta ggggaacttc ctttttccct tattgaatct ttgtcagaaa ctaaagtctt   120
   tgcaattgac agaacctata actttttttt taatataaaa gatatccaca catcactaca   180
   tgagaagcgc cttagctaat tactactgtg gtctgtgttt aaatactaaa aatgtatctg   240
   tatgactagt ttaaacaatt attcaaagag gacagtactg catgtgagct tagatctgta   300
   cttttttatg tttaggcgta agggttcaga aatatggcca ggtctagtga agaagcaagg   360
   aggattatgt atttcatttt gcattcataa accctacagc cctaaaattc ttatattgta   420
   cataaccttg gggtttgttt aaaagccact gcgacgtaaa ggagcattgt ttatcctcat   480
   gaaatcttga cctttcttag gtgtcaggcc cttgaaagga aaaattctgc aattccatca   540
   gagttgaatg aaaagcaaga gcttgtttat actaacaaaa agttagagct acaagtggaa   600
   agcatgctat cagaaatcaa aatggaacag gctaaaacag aggatgaaaa gtgagtatgt   660
   tgagtcagaa gggcagcgac ggggcagagg agggagaatc gcctttttat acagattgga   720
   attcggattt gagaataaat tttaaaaaat ttctttttca cttatctgaa ggagtcctag   780
   cagacctctc agagaggggg ataaaattta aaagttttgt cataataaaa ttatgctgat   840
   tgtttgcact ctgtcttgat ttttcagaaa agattttttt tgagagtaag aaatgctagt   900
   aggtcgtggg gtgataaagg taggcgagaa gatttttcta ctggagtgtt cagaaggttg   960
   ggaggcaaga ctataagttt ctatgatatt ttccccagga ttccattttt taatatcttt  1020
   tttaataggt ccaaattaac tgtgctacag atgacacaca acaagcttct tcaagaacat  1080
   aataatgcat tgaaaacaat tgaggaacta acaagaaaag aggtattcac tgaaaaaaat  1140
   tacttccata                                                         1150
<210> 10
   <211> 1094
   <212> DNA
   <213> Homo sapiens
<400> 10
   gcaattccat cagagttgaa tgaaaagcaa gagcttgttt atactaacaa aaagttagag      60
   ctacaagtgg aaagcatgct atcagaaatc aaaatggaac aggctaaaac agaggatgaa     120
   aagtgagtat gttgagtcag aagggcagcg acggggcaga ggagggagaa tcgccttttt     180
   atacagattg gaattcggat ttgagaataa attttaaaaa atttcttttt cacttatctg     240
   aaggagtcct agcagacctc tcagagaggg ggataaaatt taaaagtttt gtcataataa     300
   aattatgctg attgtttgca ctctgtcttg atttttcaga aaagattttt tttgagagta     360
   agaaatgcta gtaggtcgtg gggtgataaa ggtaggcgag aagatttttc tactggagtg     420
   ttcagaaggt tgggaggcaa gactataagt ttctatgata ttttccccag gattccattt     480
   tttaatatct tttttaatag gtccaaatta actgtgctac agatgacaca caacaagctt     540
   cttcaagaac ataataatgc attgaaaaca attgaggaac taacaagaaa agaggtattc     600
   actgaaaaaa attacttcca tagcctagta atgaacagaa actgttgaac gttttgtata     660
   taaaatagtt acatgaatcc ttcactaaat ctggtttcaa aggttgtttt ccaatgtatc     720
   attatttctt gcatctaggg tttgtaactt ctgatgttcc acatatgtgt aatgtgcttt     780
   attgcgtaca aagatgatgt gaatgtccta tggtcaggga ttaagcactt cgtatttctt     840
   tttttttttt tttgagacgg agtctcgctc tgtcgcccag gctggagtgc agtggcgcga     900
   tctcggctca ctgcaagctc cgcctcctgg gttcacgcca ttctcctgcc tcagcctccc     960
   gagtagctgg gactacaggc gcccgccacc gcgcccggct aattttttgt atttttagta       1020
   gagacggggt ttcaccttgt tagccaggat ggtctcgatc tcctgacctc gtgatccacc    1080
   cgcctcggcc tccc                                                     1094
<210> 11
   <211> 1159
   <212> DNA
   <213> Homo sapiens
<400> 11
   gtgctgggat tacaggtgtg agccatcatg cccagcagta gtgttcctct cttggaccta 60
   ataattttaa atttaaaaca tgtttcttct tttccactga ctgcaggaag taacaagtgg 120
   caaaataaca gtatcaacga gtcacagcct tattaacatt ggagtttgtt attgtatccc 180
   tgatttcggt gttatcacct tttttttagg aattcattat ttgcaagcca caacttaaat 240
   acaactttct gaataagtta gcgttgctga ttaatagact ggttagagct gatacatttt 300
   ttagatctcg ctatgttgcc caggcttgtc tcccactcct gggctcaaac gatcctccca 360
   cctcagcctc tcaattctag gcatgagcca ccacacccgg ccagagctga taattaaaaa 420
   aataaacctt tttctaatat tttactaaaa caggcagaat tatttcaaaa ccatttctag 480
   aataaatgtt tctttttcag tcagaaaaag tggacagggc agtgctgaag gaactgagtg 540
   aaaaactgga actggcagag aaggctctgg cttccaaaca gctgcaaatg gatgaaatga 600
   agcaaaccat tgccaagcag gaagaggacc tggaaaccat gaccatcctc agggctcagg 660
   tgaggcacct tccaaaaccc cagctgagcg aggccagccc tgactgtatt ctcgcattgg 720
   aaagcaatgg tgtttagaat gtttgtaatt ttctatttta tatatttttt cacccgtgag 780
   tgtattaaaa ctttaaaatt gaaacatttg gaaagtgctc agtggatctt atctgttcta 840
   catttaatag gtaattggat tctttccagt ttgtggcatt atgattaacg ttgctaagac 900
   attcctgtgc atgttgctct gttcacatgt ggatatttta tatttctgtt gggtacacac 960
   ctaggagtgg agtcgctgga tcataggctc tgcatgttac tcacttttaa caggtaatgc 1020
   caaacagttt tccagagtgg ttggaccagt tttcactccc atcaacagag agtttccatg 1080
   gctctacatc ttaccaacac ttctattatc agtcattttc ctttaaccac tctggagggt 1140
   atatagtggt atctcattt 1159
<210> 12
   <211> 1131
   <212> DNA
   <213> Homo sapiens
<400> 12
   tttcataagg taaaataaga tagtaaatgt aaagcaccca acataggacc tcacacatgt     60
   ttggaattta acaaatagca tctatttgtg atgattattc ttttaaattt agcttaagac    120
   cagccttcat aaatacacct ggcagaatca atttactata ttaagtaatc atttactata    180
   ttaagttgat cctgaattgt ttattatcta aaagtccaga taattttgct gaattaatgg    240
   tacctacagt atttaaacta cctatatcag tgcagttgca ggatttgtgt tgtttaaagc    300
   acacacacaa acacagcttg tatctgctat cggaatgtac ctggaaagtc atggtcatta    360
   tactgttttc tagcaggatt gtgcatctgt gattcacaag ggctattgaa ggatacagca    420
   ctacctcctc atcgcataaa cactgtaaga atctgcattc atctaggtac taacttctgt    480
   atcttttttt cctctaacag atggaagttt actgttctga ttttcatgct gaaagagcag    540
   cgagagagaa aattcatgag gaaaaggagc aactggcatt gcagctggca gttctgctga    600
   aagagaatga tgctttcgaa gacggaggca ggtaaggaaa agagagagga ggacccagag    660
   ctcacatcag catggccgta gaagaggtgc ctgtccaaag acgttcctga tttgaactat    720
   aagaatagct gtgttcgcgc cactgcactc ctgcctaggt gacagagcga gtcccctgtc    780
   tgaaaaataa ataataataa taataattgc ttcacttaca cttcatgtga tcatgttccc    840
   aacacttagt ttgtcttaca ggaaagcttg acagagactt gtgggagctt gatcaagctc    900
   cttgctttta gataagcaag gattttgatt tgattttaaa atgttgtgtt gttttgtttt    960
   gttttttgag gcagggtctc actcctgtca cccaggctgg agtgcagtgg catgatcatg    1020
   gttcactgca gcctcaactt cctgagctca ggtgatcctc gtgcctcagc ctcccgagta   1080
   gctggaacta caagtgcatg ccaccatgca cttgtaacaa taatgttacg t            1131
<210> 13
   <211> 1080
   <212> DNA
   <213> Homo sapiens
<400> 13
   accttgtgct gttaggaatt tggtgggtag cttccccatc tattttatac ttttacatat     60
   cacatacaca cttacctata tcatatctca aaaccagata atattgattt ctctgtgttt    120
   aagttacaaa atgatcactg taggtattgt tctgcagctt actttacata atattatgat    180
   tttgagctct cttgatatgt gcggatgtaa tttattatac ttcattgctg tattttgatt    240
   tataaatatg ccacttcttt ctaatctgtt tcctactgat gacagtttgg ttatttcctg    300
   atttttttta actgtaatta tttactttca ctagtctcct agtgccaata gtatttaaaa    360
   ctaaaattag tctggttttt atgaaccttg gcagtgtagt ttgagtcttt tttcccctac    420
   ttctgtggac tgtctgctca gtgttgtcat gtttcggggt tgtagaacat cacacagcgt 480
   gttgcttttc gtcctggcag gcagtccttg atggagatgc agagtcgtca tggggcgaga 540
   acaagtgact ctgaccagca ggcttacctt gttcaaagag gtgagtcccg tgtgatcctg 600
   gattttcagg aaatagctat cctatgaaaa agatgcttga agaaaaattc cacttcattc 660
   tctacaatgg attccaaatc aaggcaccaa aaatatagca cccgtcagtc tcattaccac 720
   agcactccca tctccatcca ttacccaccg aatccagacc agacccttca ccctgccaga 780
   aggtgcctgg cacggccaca ctttttcttt tttttctttt tttttgagac agaatttcgc 840
   tgtgtcgtcc aggctggagt gcagtggcga gatctcggct cactgcaacc tccacttcct 900
   gtgttcaaac ggttctcctt ccacagcctc ccgagtggct ggaattacag gcgtgcaccg 960
   ccacacccag ctaatttttg tatttttaat agagatgggg tttcaccgtg ttggccaggc 1020
   tggtctcgaa ctcctgacct caactaacct gcctgtctcg gtctcccaaa gtaccgggat 1080
<210> 14
   <211> 1122
   <212> DNA
   <213> Homo sapiens
<400> 14
   catgccagta atcctagcac tttgggaggc caaggtgggc agatcatgag gtcaggagtt     60
   cgagaccagt ctggccaaca tggcaaaacc acatctctac taaaaataca aaaattagct    120
   gggcgtggtg gcgcgcacct gtgatcccag ctactcagga ggccaaagca ggaggatcac    180
   ttgaacctgg gaggcggagg ttgcagtgag ccaagatcgt gccactgccc tccagcctgg    240
   gtgacagcga gactccgtct caaaaaaaaa aaaaaaaaaa aaaaatccta aaataatagg    300
   gaagcaggta tcacttggag agatttttct ctatgtgcat cgtgatgact tcagttaaag    360
   accaaacacc tgtgctcatg tcccactacg tgttgaatac gaagttgaac tgatgttaaa    420
   actcgccatc tgttcttcaa gtgaaacaaa cacaactgcc tgcaaaatgg aactaatgga    480
   attatcatac ttattcccag gagctgagga cagggactgg cggcaacagc ggaatattcc    540
   gattcattcc tgccccaagt gtggagaggt tctgcctgac atagacacgt tacagattca    600
   cgtgatggat tgcatcattt aagtgttgat gtatcacctc cccaaaactg ttggtaaatg    660
   tcagattttt tcctccaaga gttgtgcttt tgtgttattt gttttcactc aaatattttg    720
   cctcattatt cttgttttaa aagaaagaaa acaggccggg cacagtggct catgcctgta    780
   atcccagcac tttgggaggt cgaggtgggt ggatcacttg gggtcagggt ttgagaccag    840
   cctggccaac atggcggaac cctgtctcta ccaaaattac aaaaattagc cgagcatggt    900
   ggcgcatgcc tgtagtcgca gctactcgcg aggttgaggc aggagaattg cttgaaccca    960
   ggaagtggca gttgcagtga gccgagacga caccactgca ctccagcctg ggtgacagag   1020
   ggagactctg tctcgaaaga aagaaagaaa aaaaggaagg aaggagaagg aaggaaggag   1080
   aagaaaaggt acctgttcta cgtagaacac ctttggtgga gt                      1122
<210> 15
   <211> 23
   <212> DNA
   <213> Artificial
<220>
   <223> Designed DNA based on OPTN gene
<400> 15
   aaggaagaat caaaaatgtc caa 23
<210> 16
   <211> 25
   <212> DNA
   <213> Artificial
<220>
   <223> Designed DNA based on OPTN gene
<400> 16
   acctgtttcc aaaagtaaaa atcag 25
<210> 17
   <211> 20
   <212> DNA
   <213> Artificial
<220>
   <223> Designed DNA based on OPTN gene
<400> 17
   gaaatgatgt tcatcccgct 20
<210> 18
   <211> 21
   <212> DNA
   <213> Artificial
<220>
   <223> Designed DNA based on OPTN gene
<400> 18
   cccttaatag ggcaaccaat c 21
<210> 19
   <211> 20
   <212> DNA
   <213> Artificial
<220>
   <223> Designed DNA based on OPTN gene
<400> 19
   tgtgccacta cacctggcta 20
<210> 20
   <211> 26
   <212> DNA
   <213> Artificial
<220>
   <223> Designed DNA based on OPTN gene
<400> 20
   tttttccatt ttagctatga taaccc 26
<210> 21
   <211> 20
   <212> DNA
   <213> Artificial
<220>
   <223> Designed DNA based on OPTN gene
<400> 21
   gaaactgacc ttcacgcctt 20
<210> 22
   <211> 20
   <212> DNA
   <213> Artificial
<220>
   <223> Designed DNA based on OPTN gene
<400> 22
   gagccaaaca ggaaccaaac 20
<210> 23
   <211> 19
   <212> DNA
   <213> Artificial
<220>
   <223> Designed DNA based on OPTN gene
<400> 23
   aggaccagct gtgcttgtt 19
<210> 24
   <211> 22
   <212> DNA
   <213> Artificial
<220>
   <223> Designed DNA based on OPTN gene
<400> 24
   gctacgttgt agatgaacct ca 22
<210> 25
   <211> 20
   <212> DNA
   <213> Artificial
<220>
   <223> Designed DNA based on OPTN gene
<400> 25
   tgcatggaaa caaatggtgt 20
<210> 26
   <211> 20
   <212> DNA
   <213> Artificial
<220>
   <223> Designed DNA based on OPTN gene
<400> 26
   cacagagcag gacaaggaca 20
<210> 27
   <211> 19
   <212> DNA
   <213> Artificial
<220>
   <223> Designed DNA based on OPTN gene
<400> 27
   cccagtgcat ccaaattga 19
<210> 28
   <211> 22
   <212> DNA
   <213> Artificial
<220>
   <223> Designed DNA based on OPTN gene
<400> 28
   tcatgctcac acattaactg ga 22
<210> 29
   <211> 20
   <212> DNA
   <213> Artificial
<220>
   <223> Designed DNA based on OPTN gene
<400> 29
   ttcagaaata tggccaggtc 20
<210> 30
   <211> 20
   <212> DNA
   <213> Artificial
<220>
   <223> Designed DNA based on OPTN gene
<400> 30
   cagagtgcaa acaatcagca 20
<210> 31
   <211> 27
   <212> DNA
   <213> Artificial
<220>
   <223> Designed DNA based on OPTN gene
<400> 31
   gagagtaaga aatgctagta ggtcgtg 27
<210> 32
   <211> 21
   <212> DNA
   <213> Artificial
<220>
   <223> Designed DNA based on OPTN gene
<400> 32
   tccctgacca taggacattc a 21
<210> 33
   <211> 18
   <212> DNA
   <213> Artificial
<220>
   <223> Designed DNA based on OPTN gene
<400> 33
   ccactcctgg gctcaaac 18
<210> 34
   <211> 21
   <212> DNA
   <213> Artificial
<220>
   <223> Designed DNA based on OPTN gene
<400> 34
   tgccacaaac tggaaagaat c 21
<210> 35
   <211> 20
   <212> DNA
   <213> Artificial
<220>
   <223> Designed DNA based on OPTN gene
<400> 35
   cagtgcagtt gcaggatttg 20
<210> 36
   <211> 21
   <212> DNA
   <213> Artificial
<220>
   <223> Designed DNA based on OPTN gene
<400> 36
   tgatcaagct cccacaagtc t 21
<210> 37
   <211> 24
   <212> DNA
   <213> Artificial
<220>
   <223> Designed DNA based on OPTN gene
<400> 37
   tttcactagt ctcctagtgc caat 24
<210> 38
   <211> 20
   <212> DNA
   <213> Artificial
<220>
   <223> Designed DNA based on OPTN gene
<400> 38
   gattcggtgg gtaatggatg 20
<210> 39
   <211> 20
   <212> DNA
   <213> Artificial
<220>
   <223> Designed DNA based on OPTN gene
<400> 39
   gggaagcagg tatcacttgg 20
<210> 40
   <211> 18
   <212> DNA
   <213> Artificial
<220>
   <223> Designed DNA based on OPTN gene
<400> 40
   atccacccac ctcgacct 18

## Description

### BACKGROUND OF THE INVENTION

### 1. Field of the Invention

The present invention relates to an assay method for a glaucoma-related gene in the clinical testing field, and to an assay method for predicting a glaucoma onset risk using a mutation(s) in the gene as a marker. More specifically, the invention relates to a gene assay method in which, for example, a mutation of the gene encoding optineurin (hereinafter referred to as "OPTN") known as a glaucoma-related gene, is detected. Glaucoma is thereby predicted using, as a marker, the detected anomaly, i.e., a mutation of a base or bases at a specific position or specific positions in the gene. More particularly, the present invention relates to an assay method for predicting the possibility of a future onset of glaucoma in an individual.

### 2. Description of the Related Art

Glaucoma is a disease in which it is difficult to discharge aqueous humor from the eye and as a result ocular tension increases, resulting in a degradation of the functions of the eye. If a glaucomatous eye is left untreated, the field of vision of the eye becomes smaller or eyesight is degraded, eventually resulting in loss of eyesight. There are also cases where the ocular tension is normal but the optic nerve is damaged.

Glaucoma is classified in five disease types such as primary open angle glaucoma (POAG), normal ocular tension glaucoma (NTG), primary closing angle glaucoma (PCAG), congenital glaucoma and secondary glaucoma. Among the 20% of glaucoma said to be genetic, POAG is found in most cases. According to a nationwide epidemiological survey conducted by Japan Oculists' Society, an incorporated body, 3.56% of the population of those who are forty years old or older are glaucoma patients.

The major risk factor of glaucoma is familial anamnesis and it is strongly suggested that the onset of glaucoma has a genetic basis. In U.S. Patent No. 5,789,169 (Nguyen et al.) filed on May 17, 1996, a gene that encodes TIGR (trabecular meshwork-induced glucocorticoid response) protein is disclosed as a glaucoma related gene. The TIGR gene is also known as the MYOC gene. The U.S. Patent No. 5,789,169 (Nguyen et al.) also discloses the cDNA sequence that encodes the TIGR protein, the protein itself, molecules bonded to the protein and the nucleic acid molecule encoding the bonded molecules. That patent further provides a method and a reagent for diagnosing glaucoma and glaucoma-related diseases, as well as other diseases such as diseases of cardiac blood vessels, immune diseases, and diseases influenced by expression and activity of said protein. A method of diagnosing glaucoma in an individual by detecting the presence of a mutation in the CYP1B1 gene, one of the glaucoma-related genes that may be used as a marker of glaucoma is also disclosed (International Patent Publication No. WO 98/36098 Al). Furthermore, Rezaie, T. et al. identified the OPTN (optinuerin) gene as causing glaucoma as a gene known as GLC1E (Science, Feb. 8, 2002; 295 (5557): 1077-9). The OPTN gene (Genbank entry NM_021980) was previously known as the FIP2 gene. However, means to predict the onset risk of glaucoma in the future are not disclosed in these publications.

On the other hand, in International Patent Publication No. WO 01/88120 Al, a method of detecting a mutation at position -153 of the promoter region of the MYOC gene is disclosed. It is also described that the method can be used as a screening method for glaucoma for a patient worrying that he/she is in a family line that has a heredity risk of glaucoma, or he/she is a glaucoma carrier having no developed symptoms.

However, OPTN gene is not disclosed in the WO 01/88120A1. Since glaucoma develops slowly, a better method of early diagnosis or effectively predicting the possibility of an onset of glaucoma is desired so that measures for preventing or alleviating glaucoma can be taken before serious damage is caused to the optic nerve.

If individuals possessing a genetic risk of developing glaucoma can be identified, and examination for glaucoma can be concentrated on such individuals, it is considered that early detection and early treatment of glaucoma can be conducted efficiently. Concerning such a situation, it is the purpose of this invention to provide an assay method for genes that can be used to effectively predict the onset risk of glaucoma based upon studying the relation between glaucoma-related genes and the onset of glaucoma.

### SUMMARY OF THE INVENTION

Noting that the onset of glaucoma is related to a mutation of a gene(s), the inventors analyzed the gene sequence of the coding region of OPTN genes of glaucoma patients and non-patients and made an effort at studying them. As a result, the inventors found that the frequencies of a mutation observed for the gene(s) are significantly different between the group of patients and the group of non-patients. Furthermore, the inventors found that the onset rate of glaucoma has statistically significant dependence on whether the mutation is present or not, compared to the onset rate of the general group, and the inventors thus completed the invention.

That is, the present invention provides:
1. A gene assay method comprising the steps of:
   detecting substitution of A for G at position 898 in the coding region of a optineurin (OPTN) gene in a sample obtained from a human subject, said OPTN gene having a nucleic acid sequence denoted by SEQ ID NO: 1; and
   predicting future onset of glaucoma in the subject using the mutation as an index.
2. The gene method according to item 1, wherein the substitution is detected by using an oligonucleotide capable of forming a hybrid at a specific position of the coding region of the OPTN gene.
3. An oligonucleotide selected from the group consisting of:
   (1) an oligonucleotide represented by SEQ ID NO: 27;
   (2) a complementary chain of an oligonucleotide according to (1);
   (3) an oligonucleotide represented by SEQ ID NO: 28; and
   (4) a complementary chain of an oligonucleotide according to (3).
4. A gene assay method for predicting future onset of glaucoma, comprising steps of :
   (a) isolating a polynucleotide from a sample obtained from a human subject;
   (b) performing a nucleic acid amplification process on said polynucleotide using an oligonucleotide of item 3;
   (c) detecting a substitution of A for G at position 898 in the coding region of a glaucoma-related gene having a nucleic acid sequence denoted by SEQ ID NO: 1; and
   (d) predicting future onset of glaucoma in the subject using the mutation as an index.
5. The gene assay method according to item 1 or 4, wherein the glaucoma is primary open angle glaucoma and/or normal ocular tension glaucoma.
6. An assaying reagent or an assaying reagent kit comprising an oligonucleotide of item 3.
7. The gene assay method according to claim 1, wherein the glaucoma is primary open angle glaucoma and/or normal ocular tension glaucoma.
8. The gene assay method according to item 1, wherein the mutation is detected by using an oligonucleotide capable of forming a hybrid at a specific position of the coding region of the OPTN gene.
9. An oligonucleotide selected from the group consisting of
   (1) an oligonucleotide consisting of a base sequence represented by SEQ ID NOs: 27 and 28
   (2) a complementary chain of an oligonucleotide according to (1).
10. A gene assay method for predicting future onset of primary open angle glaucoma and/or normal ocular tension glaucoma, comprising the steps of:
   (a) isolating a polynucleotide sample from a subject suspected of having a mutation in a glaucoma-related gene,
   (b) performing a nucleic acid amplification process on said polynucleotide using at least one oligonucleotide selected from the group consisting of:
      (1) an oligonucleotide consisting of a base sequence represented by SEQ ID NOs: 27 and 28;
      (2) a complementary chain of an oligonucleotide according to (1);
   (c) detecting a mutation of at least one base in the coding region of a glaucoma-related gene; and
   (d) predicting future onset of primary open angle glaucoma and/or normal ocular tension glaucoma using the mutation as an index.
11. An assaying reagent or an assaying reagent kit comprising an oligonucleotide of item 9.

### BRIEF DESCRIPTION OF THE DRAWINGS

Fig. 1 shows the structure of the OPTN gene (Example 1);
Fig. 2 shows the structure of exon 4 of the OPTN gene and the relation of positions between it and its corresponding primer (Example 1);
Fig. 3 shows the structure of exon 5 of the OPTN gene and the relation of positions between it and its corresponding primer (Example 1);
Fig. 4 shows the structure of exon 6 of the OPTN gene and the relation of positions between it and its corresponding primer (Example 1);
Fig. 5 shows the structure of exon 7 of the OPTN gene and the relation of positions between it and its corresponding primer (Example 1);
Fig. 6 shows the structure of exon 8 of the OPTN gene and the relation of positions between it and its corresponding primer (Example 1);
Fig. 7 shows the structure of exon 9 of the OPTN gene and the relation of positions between it and its corresponding primer (Example 1);
Fig. 8 shows the structure of exon 10 of the OPTN gene and the relation of positions between it and its corresponding primer (Example 1);
Fig. 9 shows the structure of exon 11 of the OPTN gene and the relation of positions between it and its corresponding primer (Example 1);
Fig. 10 shows the structure of exon 12 of the OPTN gene and the relation of positions between it and its corresponding primer (Example 1);
Fig. 11 shows the structure of exon 13 of the OPTN gene and the relation of positions between it and its corresponding primer (Example 1);
Fig. 12 shows the structure of exon 14 of the OPTN gene the relation of positions between it and its corresponding primer (Example 1);
Fig. 13 shows the structure of exon 15 of the OPTN gene and the relation of positions between it and its corresponding primer (Example 1); and
Fig. 14 shows the structure of exon 16 of the OPTN gene and the relation of positions between it and its corresponding primer (Example 1).

### DESCRIPTION OF THE PREFERRED EMBODIMENTS

The inventors determined the coding sequence in the coding region of each exon, constituting a glaucoma-related gene, consisting of the base sequence denoted by SEQ ID NO: 1. In the course of this determination, it was verified that genetic polymorphism occurs with a difference in frequency in the coding region of the gene between the glaucoma patient group and the non-patient group. Furthermore, the onset rate of glaucoma depends thereon statistically in a significant manner compared to the rate thereof in the general group. The invention is constituted based on the above new findings.

### [Glaucoma-related Genes]

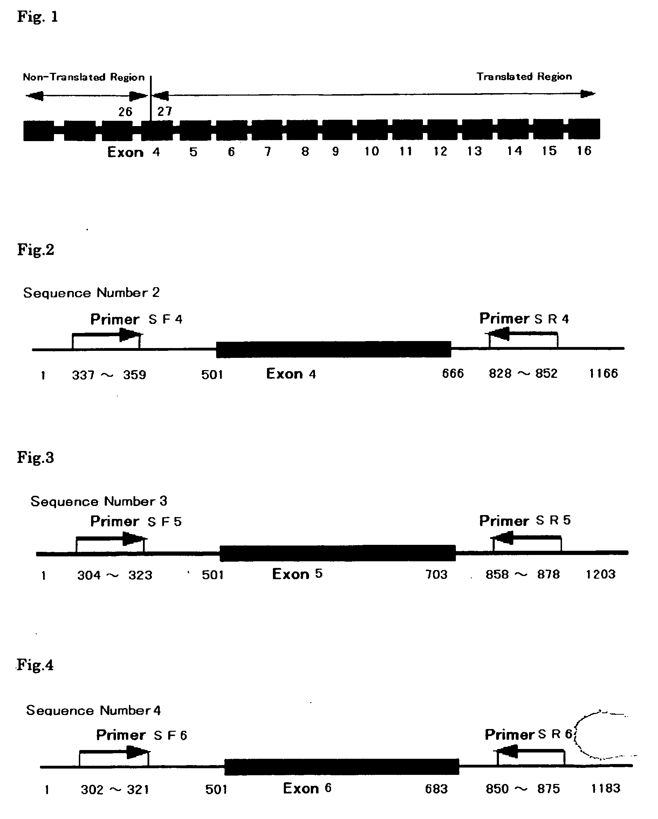

As an example of the glaucoma-related genes, OPTN (optineurin) gene can be noted. The structure and the coding sequence of the optineurin gene are as shown in Fig. 1 and as listed in Table 1 and, for example, there are coding regions to be transcribed and translated into protein, and non-translated regions as well as other elements. The base positions of the OPTN gene conform with the base numbers defined in SEQ ID NO: 1 (Genbank entry number AF420371, AF420372 and AF420373). The OPTN protein contains 13 exons. The sequence of each exon and the location of it is denoted by SEQ ID NO: 2 for exon 4, by SEQ ID NO: 3 for exon 5, by SEQ ID NO: 4 for exon 6, by SEQ ID NO: 5 for exon 7, by SEQ ID NO: 6 for exon 8, by SEQ ID NO: 7 for exon 9, by SEQ ID NO: 8 for exon 10, by SEQ ID NO: 9 for exon 11, by SEQ ID NO: 10 for exon 12, by SEQ ID NO: 11 for exon 13, by SEQ ID NO: 12 for exon 14, by SEQ ID NO: 13 for exon 15 and by SEQ ID NO: 14 for exon 16. The correspondence between the base sequence of the OPTN gene represented as the sequence denoted by SEQ ID NO: 1 and the base sequence of each exon represented as the sequences denoted by SEQ ID NOS: 2 to 14 is as follows. The positions 501-666 in SEQ ID NO: 2 to the positions 1-166 of SEQ ID NO: 1, the positions 501-703 in SEQ ID NO: 3 to the positions 167-369 of SEQ ID NO: 1, the positions 501-683 in SEQ ID NO: 4 to the positions 370-552 of SEQ ID NO: 1, the positions 501-574 in SEQ ID NO: 5 to the positions 553-626 of SEQ ID NO: 1, the positions 501-653 in SEQ ID NO: 6 to the positions 627-779 of SEQ ID NO: 1, the positions 501-603 in SEQ ID NO: 7 to the positions 780-882 of SEQ ID NO: 1, the positions 501-616 in SEQ ID NO: 8 to the positions 883-998 of SEQ ID NO: 1, the positions 501-650 in SEQ ID NO: 9 to the positions 999-1148 of SEQ ID NO: 1, the positions 501-594 in SEQ ID NO: 10 to the positions 1149-1242 of SEQ ID NO: 1, the positions 501-659 in SEQ ID NO: 11 to the positions 1243-1401 of SEQ ID NO: 1, the positions 501-631 in SEQ ID NO: 12 to the positions 1402-1532 of SEQ ID NO: 1, the positions 501-580 in SEQ ID NO: 13 to the positions 1533-1612 of SEQ ID NO: 1, the positions 501-622 in SEQ ID NO: 14 to the positions 1613-1734 of SEQ ID NO: 1.

### [Gene mutation]

A mutation in the glaucoma-related gene for detection in the present invention refers to substitution, of one base at a specific position(s) in the base sequence of the OPTN gene. The "specific position" refers to a position 898 of the base sequence denoted by SEQ ID NO: 1.

As the detailed substitution of bases at specific positions in the invention, a substitution of A for G at the position 898 in the base sequence denoted by SEQ ID NO: 1 are mentioned.

### [Assay Method]

In the method for assaying for the mutation of the glaucoma-related gene, the method is not limited, as far as detecting the specific mutation of the OPTN gene disclosed herein, but various methods currently known or to be known in the future can be used for detection of a relevant mutation.

In order to check for a mutation as disclosed in this invention in the OPTN gene of a subject, various methods can be used for analyzing the base sequences containing the position(s) of a possible mutation. As these methods, for example, Southern hybridization method, dot hybridization method (see J. Mol. Biol, 98: 503-517 (1975) etc.), dideoxy base sequence determination method (Sanger's method), the various detecting methods combined with DNA amplification approaches can be listed [for example, PCR-restriction fragments length polymorphism analyzing method (RFLP), PCR-single-chain-high-order structure polymorphism analyzing method (see Proc. Natl. Acad. Sci., U.S.A., 86: 2766-2770 (1989) etc.), PCR-specific sequence oligonucleotide method (SSO), allele specific oligonucleotide method using PCR-SSO and dot hybridization method (see Nature, 324: 163-166 (1986) etc.)] can be employed. For the position(s) of the gene mutation to be detected as disclosed and identified in this invention, those skilled in the art can detect the mutation using known methods.

### [Preparation of the Sample for Measurement]

In order to analyze the OPTN gene of a subject, the sample to be prepared for the assay method of the invention is not specifically limited but may be any biological sample containing the OPTN gene of the subject, such as tissues collected from a living body, tissue cut out in an operation and tissue from the oral cavity mucous membrane, blood, serum, excretions, ejaculated semen, expectorated sputum, saliva, brain and spinal fluid, hair etc. For example, the OPTN gene extracted by a known gene extraction method such as phenol-chloroform method, from a biological sample such as tissue that has been crushed using a blender can be used as a sample to be examined. Furthermore, the extracted OPTN gene can be prepared as a sample to be examined by amplifying and concentrating it.

The sample to be examined may either be the full-length of the DNA of the OPTN gene or a DNA fragment (a portion of the full-length DNA). When a DNA fragment is examined, it is preferred for the fragment to include the complete or partial coding region of at least one (1), preferably, two (2) or more, and, more preferably, three (3) or more exons. There is no limitation on the DNA fragment in terms of its base length as long as it is useable for the detection of a gene mutation, i.e., as long as it has a length available for measurement as a sample DNA prepared for the examination of a base mutation. As the base length of such DNA, a length of around ten (10) or more bases and, preferably, around 20 or more bases can be selected. Generally, a length of around 100-1000 bases and, preferably, around 200-300 bases is selected.

Furthermore, the sample to be examined may either be DNA or a DNA transcription product. More specifically, the sample may either be a messenger RNA (mRNA) transcribed from DNA, cDNA further reverse-transcribed from the mRNA or other complementary DNA. All of the various steps that may be employed in the detection method for the gene mutation of the invention, such as, for example, synthesis of DNA or DNA fragment; enzyme treatments with the purpose of cutting, deleting, adding or coupling DNA; isolation, purification, duplication and selection of DNA; and amplification of DNA fragment may be performed according to conventional methods (see Methods for Experiments in Molecular Genetics, Kyouritsu Shuppan Co., Ltd., 1983, etc.). These steps can be modified according to conventional procedure as necessary.

Amplification of nucleic acid in order to prepare a sample to be examined may be implemented according to, for example, a PCR method or its variant method (see PCR Technology, Takara Shuzo Co., Ltd., 1990, etc.). In this case, an oligonucleotide capable of specifically forming a hybrid at a portion of a glaucoma-related gene, more specifically, an oligonucleotide having a primer function, is suitably selected so that it specifically amplifies a desired DNA fragment having at least one (1) or more of the above specific positions, related to mutation.

### [Oliginucleotide Having a Primer Function]

As oligonucleotides having a primer function, an oligonucleotide such as, for example, (1) an oligonucleotide comprising a base sequence denoted by SEQ ID NO:s 27 or 28, (2) a complementary chain of an oligonucleotide described in above (1), (3) can be used.

An oligonucleotide itself can be designed by known methods, i.e., for example, it can be chemically synthesized. Also, it is possible to cut natural chain(s) of nucleic acid using restriction enzyme etc., to modify the natural nucleic acid chain or to couple or cut chains so that the natural nucleic acid is constituted by an above base sequence. More specifically, oligonucleotides can be synthesized using an oligonucleotide synthesizing apparatus (manufactured by Applied Biosystems, Expedite Model 8909 DNA Synthesizer) etc. Furthermore, known production methods can be used as synthesizing methods of oligonucleotides having one (1) to several bases varied such as by substitution, deletion, insertion or addition. The synthesis can be carried out using, for example, portion-specific mutation introduction method, gene homologous recombination method, primer elongation method and polymerase chain reaction method (PCR) or using a plurality of these methods in combination. Furthermore, the synthesis can be carried out according to the methods described in, for example, Molecular Cloning; A Laboratory Manual, Second Edition, Edited by Sambrook et al., Cold Spring Harbor Laboratory Press, Cold Spring Harbor, New York, 1989; Labo Manual Genetic Engineering, Edited by Masami Matsumura, Maruzen Co., Ltd., 1988; PCR Technology, The Principle and Application of DNA Amplification, Edited by Ehrlich, HE., Stockton Press, 1989, etc. Furthermore, a modified version of an above method such as, for example, a technique by Ulmer (Science (1983) 219:666) can be used.

Generally known conditions can be selected as the stringent conditions for hybridization and, as an example thereof, , after hybridizing over night at 42°C in a solution containing 50%-formamide, 5X SSC (150mM of NaCl, 15mM of trisodium citrate), 50mM of sodium phosphate (pH 7.6), 5X Denhart's solution, 10%-dextran sulfuric acid, and 20µg/ml of DNA, a primary washing in 2X SSC•0.1% of SDS at room temperature and a secondary washing in 0.1 X SSC•0.1% of SDS.

### [Detection of a mutation of Gene DNA]

A mutation of DNA can be detected by, for example, determining the base sequence of the OPTN gene contained in the sample to be examined using Sanger's method.

Using a one-chain target OPTN gene hybridized with a complementary oligonucleotide as a primer, a complementary chain is synthesized with DNA polymerase in the direction from the position 5' to the position 3'. The oligonucleotide used as a primer is, for example, an oligonucleotide as described above.

A complementary chain is synthesized by adding a small amount of dideoxyribonucleotide-triphosphoric acid (ddNTP) to each base in addition to four (4) kinds of deoxyribonucleotide-triphosphoric acid (dNTP) as substrates for the reaction. ddNTP is an analogue (analogous substance) having an -H group at the position 3' of a deoxyribose instead of an -OH group. Once ddNTP is taken in instead of dNTP, the complementary chain is not synthesized further and DNAs having various lengths are obtained. In the reaction system, the synthesized DNA can be labeled by adding, for example, a primer or dNTP labeled with a chemiluminescent substance or a radioactive isotope. Then, the base sequence can be determined by the electrophoresis of reaction products with denatured polyacrylamide.

For example, Klenow enzyme, T7 phage and thermophilic-bacteria-originated DNA polymerases can be listed as DNA polymerase used in Sanger's method. The exonuclease activity of these DNA polymerases is commonly removed in a genetic-engineering approach. At first, the target genes were used in a form of one chain in Sanger's method but, at present, a method is often used in which double-chain plasmid is alkali-denatured as it is and is used in the detection method.

Sequence reactions can be carried out by Sanger's method or a cycle sequence method. Cycle sequence method is a method formed by combining Sanger's method and the PCR method. In this method, template DNA does not need to be single-chain DNA. The reaction is carried out with DNA, one kind of primer, dNTPs, ddNTPs and heat-resistant DNA polymerase in the reaction system. It is the same as Sanger's method in that, during the PCR reaction, ddNTPs are taken up, elongation is terminated and, as a result, DNA having the same bases at the 3'-terminal position is synthesized. As sequence reactions for automatic sequencers, there are dye primer method in which the primer is marked with fluophor, dye terminator method in which ddNTP is marked with fluophor, internal-label method in which dNTP substrate is labeled etc.

### [Assaying Reagent and Assaying Reagent Kit]

The invention also includes an assaying reagent and an assaying reagent kit used for the gene assay method for predicting glaucoma. The assaying reagent may be any of the various reagents used for the method of the invention such as, for example, a primer for amplification of the sample to be examined, a primer for determining the base sequences of the sample to be examined, various polymerase, base substrates, marking materials, etc. The assaying reagent kit may be any kit in which at least two (2) of the reagents used for the method of the invention are present.

### [Examples]

The invention will be specifically described referring to examples. However, the invention is not limited to those examples.

### [Example 1]

DNA Analysis of OPTN Gene

### (1) Extraction of DNA

Blood provided by a subject was processed according to a conventional method and DNA was extracted from an eukaryotic cell. Using a kit product named "Dr. GenTLE^{™} (for blood) (manufactured by Takara Shuzo Co., Ltd.,)" as the DNA extraction kit, DNA was extracted according to the protocol provided by the instruction manual of the product.

### (2) Amplification of Template DNA

Using the obtained DNA extraction solution as a template, OPTN gene was amplified in PCR using a kit product named "LATaq (manufactured by Applied Biosystems)", a kit for PCR amplification.

The sequence of each exon from exon 4 to exon 16 is disclosed in a Genbank entry No. NT_031849 and each sequence is represented by the base sequence of the region described as follows in the sequences denoted by SEQ ID NO:s 2 to 14.

| | |
|---|---|
| Exon 4: | SEQ ID NO: 2, the positions 501-666 |
| Exon 5: | SEQ ID NO: 3, the positions 501-703 |
| Exon 6: | SEQ ID NO: 4, the positions 501-683 |
| Exon 7: | SEQ ID NO: 5, the positions 501-574 |
| Exon 8: | SEQ ID NO: 6, the positions 501-653 |
| Exon 9: | SEQ ID NO: 7, the positions 501-603 |
| Exon 10: | SEQ ID NO: 8, the positions 501-616 |
| Exon 11: | SEQ ID NO: 9, the positions 501-650 |
| Exon 12: | SEQ ID NO: 10, the positions 501-594 |
| Exon 13: | SEQ ID NO: 11, the positions 501-659 |
| Exon 14: | SEQ ID NO: 12, the positions 501-631 |
| Exon 15: | SEQ ID NO: 13, the positions 501-580 |
| Exon 16: | SEQ ID NO: 14, the positions 501-622 |

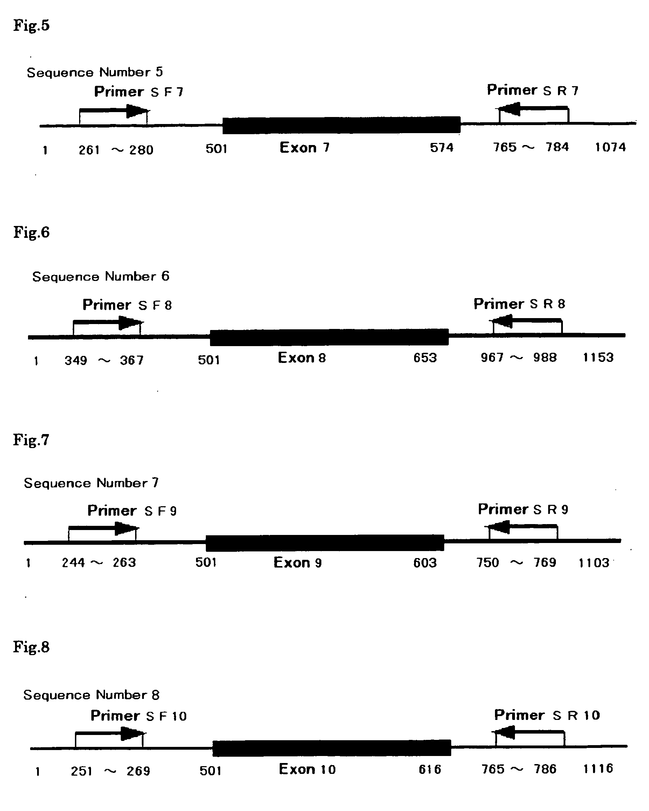

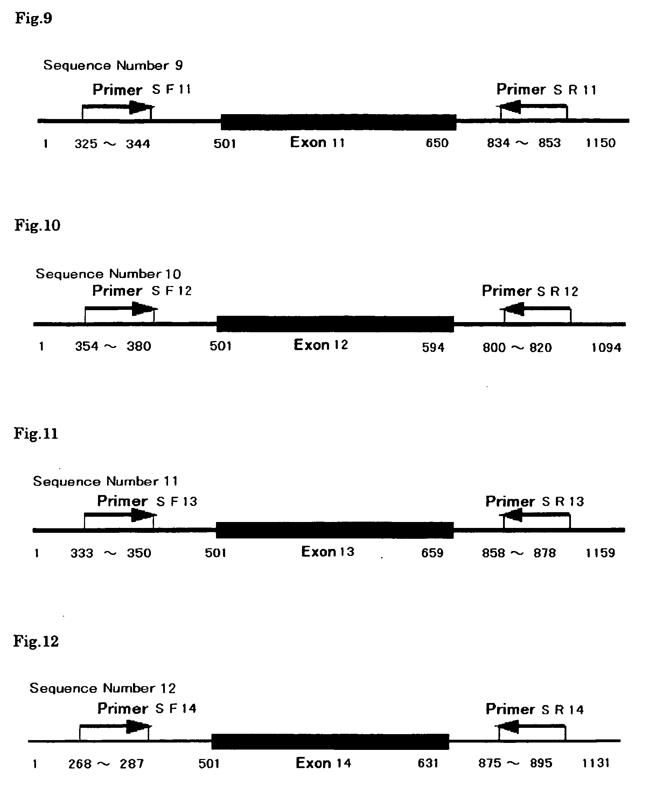

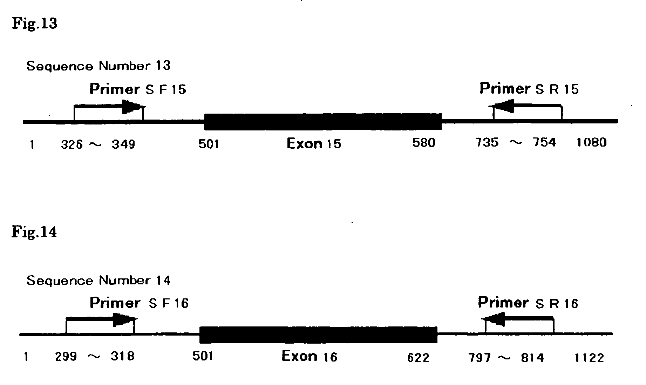

Oligonucleotide represented by each of the base sequences denoted by SEQ ID NOS: 15 to 40 was used to amplify each exon. The relation of positions between each primer and each exon is shown in Figs. 2 to 14. However, each antisense primer consists of sequences based on a complementary chain of the sequences denoted by SEQ ID NOS: 2 to 14.
Primer for Exon 4
   Normal Chain, SF4, SEQ ID NO: 15; region = SEQ ID NO: 2, the positions 337-359
   Inverted Chain, SR4, SEQ ID NO: 16; region = the complementary sequence of SEQ ID NO: 2, positions 828-852
Primer for Exon 5
   Normal Chain, SF5, SEQ ID NO: 17; region = SEQ ID NO: 3, the positions 304-323
   Inverted Chain, SR5, SEQ ID NO: 18; region = the complementary sequence of SEQ ID NO: 3, positions 858-878
Primer for Exon 6
   Normal Chain, SF6, SEQ ID NO: 19; region = SEQ ID NO: 4, the positions 302-321
   Inverted Chain, SR6, SEQ ID NO: 20; region = the complementary sequence of SEQ ID NO: 4, positions 850-875
Primer for Exon 7
   Normal Chain, SF7, SEQ ID NO: 21; region = SEQ ID NO: 5, the positions 261-280
   Inverted Chain, SR7, SEQ ID NO: 22; region = the complementary sequence of SEQ ID NO: 5, positions 765-784
Primer for Exon 8
   Normal Chain, SF8, SEQ ID NO: 23; region = SEQ ID NO: 6, the positions 349-367
   Inverted Chain, SR8, SEQ ID NO: 24; region = the complementary sequence of SEQ ID NO: 6, positions 967-988
Primer for Exon 9
   Normal Chain, SF9, SEQ ID NO: 25; region = SEQ ID NO: 7, the positions 244-263
   Inverted Chain, SR9, SEQ ID NO: 26; region = the complementary sequence of SEQ ID NO: 7, positions 750-769
Primer for Exon 10
   Normal Chain, SF10, SEQ ID NO: 27; region = SEQ ID NO: 8, the positions 251-269
   Inverted Chain, SR10, SEQ ID NO: 28; region = the complementary sequence of SEQ ID NO: 8, positions 765-786
Primer for Exon 11
   Normal Chain, SF11, SEQ ID NO: 29; region = SEQ ID NO: 9, the positions 325-344
   Inverted Chain, SR11, SEQ ID NO: 30; region = the complementary sequence of SEQ ID NO: 9, positions 834-853
Primer for Exon 12
   Normal Chain, SF12, SEQ ID NO: 31; region = SEQ ID NO: 10, the positions 354-380
   Inverted Chain, SR12, SEQ ID NO: 32; region = the complementary sequence of SEQ ID NO: 10, positions 800-820
Primer for Exon 13
   Normal Chain, SF13, SEQ ID NO: 33; region = SEQ ID NO: 11, the positions 333-350
   Inverted Chain, SR13, SEQ ID NO: 34; region = the complementary sequence of SEQ ID NO: 11, positions 858-878
Primer for Exon 14
   Normal Chain, SF14, SEQ ID NO: 35; region = SEQ ID NO: 12, the positions 268-287
   Inverted Chain, SR14, SEQ ID NO: 36; region = the complementary sequence of SEQ ID NO: 12, positions 875-895
Primer for Exon 15
   Normal Chain, SF15, SEQ ID NO: 37; region = SEQ ID NO: 13, the positions 326-349
   Inverted Chain, SR15, SEQ ID NO: 38; region = the complementary sequence of SEQ ID NO: 13, positions 735-754
Primer for Exon 16
   Normal Chain, SF16, SEQ ID NO: 39; region = SEQ ID NO: 14, the positions 299-318
   Inverted Chain, SR16, SEQ ID NO: 40; region = the complementary sequence of SEQ ID NO: 14, positions 797-814

The PCR reaction was carried out such that a cycle of heating 30 seconds at 94°C, 30 seconds at 60°C and 30 seconds at 72°C was repeated for 30 times.

### (3) Determination of the Base Sequence of DNA fragment of Each Exon

The base sequence of the DNA fragment obtained by above PCR for each exon was determined using an automatic DNA sequencer ABI Prism3100 (manufactured by Applied Biosystems) according to the protocol provided by the instruction manual of the sequencer. In this procedure, the cyclic sequence reactions were carried out with either a forward primer or a reverse primer among the primers used in PCR reactions for each exon. (4) Determination of the Base Sequence of DNA fragment of Each Exon in the Control Group.

Furthermore, blood obtained from a group of non-patient volunteers as a control group was processed according to the above approach and the base sequence dominant in the non-patient group was determined for DNA fragment of each exon.

### [Example 2]

### (1) Analysis of Polymorphism of the Base Sequence of OPTN Gene

Blood obtained from a patient diagnosed to have open angle glaucoma by a medical institution was processed according to the approach used in the above Example. Then, the base sequence of the OPTN gene contained therein was studied and the sequence was compared to base sequences of the non-patient group.

Table 1 shows the result of the above. The first line in Table 1 lists exon numbers, the second line lists the SEQ ID NO:s representing each exon, the third line lists the base positions in each sequence and the fourth line lists the change of bases detected as a mutation.

As a result, a mutation was recognized only for the patient group and no mutation was recognized for the non-patient group at the base position 567 in the SEQ ID NO: 5, i.e., the base position 619 in the SEQ ID NO: 1 and at the base position 516 in the SEQ ID NO: 8, i.e., the base position 898 in the SEQ ID NO: 1.

**Table 1**

| | | |
|---|---|---|
| Exon | 7 | 10 |
| SEQ ID NO: | 5 | 8 |
| Base Position | 567 | 516 |
| Position in the SEQ ID NO: 1 | 619 | 898 |
| A mutation | A to G | G to A |
| Frequency in a patient group | 1.4% | 0.8% |
| Frequency in a nin-patient group | 0.0% | 0.0% |

As has been set forth, the disclosure obtained concerning gene mutation according to the invention is effective for predicting future onset of glaucoma. When the future onset of, especially, open angle glaucoma can be predicted by detecting a mutation of the OPTN gene by a gene assay method of the invention, it is possible to prevent the onset of the disease or to treat the disease early on.

## Claims

1. A gene assay method comprising the steps of:
detecting substitution of A for G at position 898 in the coding region of a optineurin (OPTN) gene in a sample obtained from a human subject, said OPTN gene having a nucleic acid sequence denoted by SEQ ID NO: 1; and
predicting future onset of glaucoma in the subject using the mutation as an index.

2. The gene method according to claim 1, wherein the substitution is detected by using an oligonucleotide capable of forming a hybrid at a specific position of the coding region of the OPTN gene.

3. An oligonucleotide selected from the group consisting of:
(1) an oligonucleotide represented by SEQ ID NO: 27;
(2) a complementary chain of an oligonucleotide according to (1);
(3) an oligonucleotide represented by SEQ ID NO: 28; and
(4) a complementary chain of an oligonucleotide according to (3).

4. A gene assay method for predicting future onset of glaucoma, comprising steps of :
(a) isolating a polynucleotide from a sample obtained from a human subject;
(b) performing a nucleic acid amplification process on said polynucleotide using an oligonucleotide of claim 3;
(c) detecting a substitution of A for G at position 898 in the coding region of a glaucoma-related gene having a nucleic acid sequence denoted by SEQ ID NO: 1; and
(d) predicting future onset of glaucoma in the subject using the mutation as an index.

5. The gene assay method according to claim 1 or 4, wherein the glaucoma is primary open angle glaucoma and/or normal ocular tension glaucoma.

6. An assaying reagent or an assaying reagent kit comprising an oligonucleotide of claim 3.

## Patentansprüche

1. Genassay-Verfahren, umfassend die Schritte:
Nachweisen des Ersatzes von A für G an Position 898 im codierenden Bereich eines Optineurin (OPTN)-Gens in einer Probe, die von einem menschlichen Individuum erhalten wurde, wobei das OPTN-Gen eine wie in SEQ ID NO: 1 angegebene Nucleinsäuresequenz hat; und
Vorhersagen der zukünftigen Entstehung eines Glaukoms im Individuum unter Verwendung der Mutation als Indikator.

2. Genassay-Verfahren nach Anspruch 1, wobei der Ersatz unter Verwendung eines Oligonucleotids nachgewiesen wird, das ein Hybrid an einer spezifischen Position des codierenden Bereichs des OPTN-Gens bilden kann.

3. Oligonucleotid, ausgewählt aus der Gruppe bestehend aus:
(1) einem Oligonucleotid wie in SEQ ID NO: 27 dargestellt;
(2) einer komplementären Kette eines Oligonucleotids gemäß (1);
(3) einem Oligonucleotid wie in SEQ ID NO: 28 dargestellt; und
(4) einer komplementären Kette eines Oligonucleotids gemäß (3).

4. Genassay-Verfahren zum Vorhersagen der zukünftigen Entstehung eines Glaukoms, umfassend die Schritte:
(a) Isolieren eines Polynucleotids aus einer Probe, die von einem menschlichen Individuum erhalten wurde;
(b) Durchführen eines Nucleinsäureamplifikationsverfahrens mit dem Polynucleotid unter Verwendung eines Oligonucleotids nach Anspruch 3;
(c) Nachweisen eines Ersatzes von A für G an Position 898 im codierenden Bereich eines mit Glaukom in Beziehung stehenden Gens mit einer Nucleinsäuresequenz wie in SEQ ID NO: 1 angegeben; und
(d) Vorhersagen der zukünftigen Entstehung eines Glaukoms in dem Individuum unter Verwendung der Mutation als Indikator.

5. Genassay-Verfahren nach Anspruch 1 oder 4, wobei das Glaukom ein primäres Offenwinkelglaukom und/oder ein Glaukom mit normalem Augeninnendruck ist.

6. Nachweisreagenz oder Nachweisreagenzkit umfassend das Oligonucleotid nach Anspruch 3.

## Revendications

1. Méthode d'analyse de gène comprenant les étapes :
de détection de la substitution de G par A à la position 898 dans la région codante d'un gène codant pour l'optineurine (OPTN) dans un échantillon obtenu d'un sujet humain, ledit gène OPTN ayant une séquence d'acide nucléique notée SEQ ID NO : 1 ; et
de pronostic de l'apparition future d'un glaucome chez le sujet en utilisant la mutation comme indice.

2. Méthode d'analyse de gène selon la revendication 1, dans lequel la substitution est détectée en utilisant un oligonucléotide capable de former un hybride à une position spécifique de la région codante du gène OPTN.

3. Oligonucléotide choisi dans le groupe constitué de :
(1) un oligonucléotide représenté par SEQ ID NO : 27 ;
(2) une chaîne complémentaire d'un oligonucléotide selon (1) ;
(3) un oligonucléotide représenté par SEQ ID NO : 28 ; et
(4) une chaîne complémentaire d'un oligonucléotide selon (3).

4. Méthode d'analyse de gène pour pronostiquer l'apparition future d'un glaucome, comprenant les étapes :
(a) d'isolement d'un polynucléotide à partir d'un échantillon obtenu d'un sujet humain ;
(b) de mise en oeuvre d'un procédé d'amplification d'acide nucléique sur ledit polynucléotide en utilisant un oligonucléotide de la revendication 3 ;
(c) de détection d'une substitution de G par A à la position 898 dans la région codante d'un gène lié au glaucome ayant une séquence d'acide nucléique notée SEQ ID NO : 1 ; et
(d) de pronostic de l'apparition future d'un glaucome chez le sujet en utilisant la mutation comme indice.

5. Méthode d'analyse de gène selon la revendication 1 ou 4, dans lequel le glaucome est un glaucome chronique primaire et/ou un glaucome de tension oculaire normal.

6. Réactif pour l'analyse ou kit de réactifs pour l'analyse comprenant un oligonucléotide selon la revendication 3.
